# EUROPEAN PATENT APPLICATION

(11) **EP 2 883 876 A1**
(43) Date of publication of application: **17.06.2015**
(21) Application number: 13197481.8
(22) Date of filing: 16.12.2013
(51) Int. Cl.: C07D 491/08, C07D 207/12, C07D 207/16, C07B 53/00

(54) **Stereoselective synthesis of substituted pyrrolidines**

(71) Applicant: Actelion Pharmaceuticals Ltd., 4123 Allschwil (CH)
(72) Inventor: Abele, Stefan, 4123 Allschwil (CH); Reber, Stefan, 4123 Allschwil (CH)
(74) Representative: Velker, Jörg

(57) **Abstract**

The present invention relates to a halo-lactonization process for the stereoselective preparation of protected 1-(halo-methyl)-3-oxo-2-oxa-5-azabicyclo[2.2.1]heptane derivatives of formula (II) 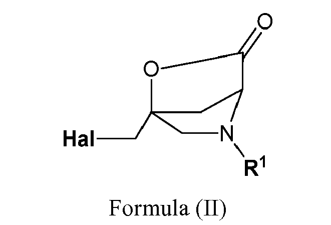 wherein Hal and R¹ are as defined in the description. The invention further relates to the novel compounds of formula (II), and to their further transformation to the compounds of formula (III) 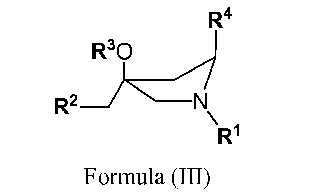 wherein R¹, R², R³, and R⁴ are as defined in the description.

## Description

The present invention relates to a new process for the stereoselective preparation of protected 1-(halo-methyl)-3-oxo-2-oxa-5-azabicyclo[2.2.1]heptane derivatives of formula (II) by a stereoselective halo-lactonization protocol starting from protected exo-methylene proline derivatives of formula (I):

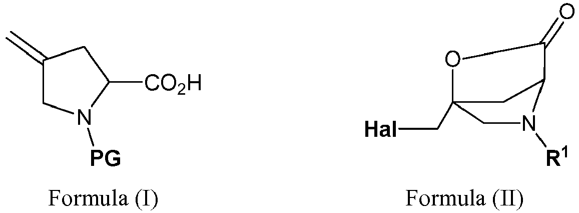

Thus, starting for example from enantiopure (*S*)-exo-methylene Boc-proline [Structure **1a**, CAS 84348-38-9], the corresponding (1*S*,4*S*)-*tert*-butyl 1-(iodomethyl)-3-oxo-2-oxa-5-azabicyclo[2.2.1]heptane-5-carboxylate (Structure **2a**) is obtained through a iodolactonization reaction:

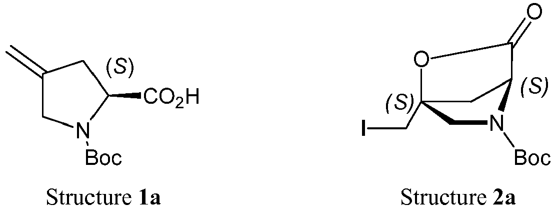

Synthetic methods to obtain the (*S*)-exo-methylene Boc-proline derivatives of formula (I) are reported in the literature. For example, the compound of structure **1a** was prepared using a standard Wittig olefination protocol (WO2008/106058, Vertex Pharmaceuticals Ltd, p.94). The Cbz-protected methyl ester derivative was prepared with a Zirconium variant of Tebbe's reagent in the presence of zinc and dibromomethane (WO2007/069020, Vicuron Pharmaceuticals Inc.).

The compounds of formula (II) can be further transformed into compounds of formula (III) which in turn can be used as versatile intermediates in the stereoselective preparation for example of pharmaceutically active compounds.

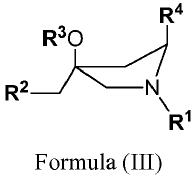

Compounds of formula (III) contain orthogonally protected functional groups and have been used for the synthesis of pharmacologically active agents. For example, the compound of structure **3a** is an intermediate for the synthesis of pharmacologically active agents for the treatment of age-related macular degeneration (WO2012/093101).

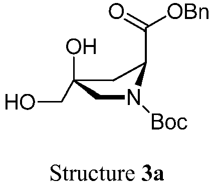

The *Sharpless* dihydroxylation protocol was used for the transformation of the 4-exo-methylene Boc-proline into the dihydroxy Boc-proline benzyl ester of structure **3a.** Impediments for larger scales are the use of the toxic osmium catalyst, the low selectivity requiring flash chromatography of the obtained diastereoisomers, and the high costs of the reagents. Similarly, compounds of structure **3b** have been obtained from 4-exo-methylene Boc-proline by *Sharpless* dihydroxylation, followed by mesylation and iodination (WO2013/052369).

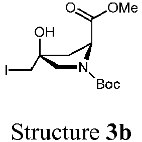

Other derivatives, conceivably prepared from compounds of formula (II) using well known experimental procedures (see for example WO2008/106058, Vertex, ,are precursors for quinoline derivatives and analogs for use in the treatment of hepatitis C virus infection. Inhibitors of serine protease (WO2008/106058, Vertex) have been prepared from *N*-Boc 4-oxo-proline *tert*-butyl ester and the zinc enolate of ethyl bromoacetate as a 4:1 mixture of diastereoisomer (in favor of the 4-(*S*) isomer), followed by a *Curtius* rearrangement. The diastereoisomers have been separated by chromatography.

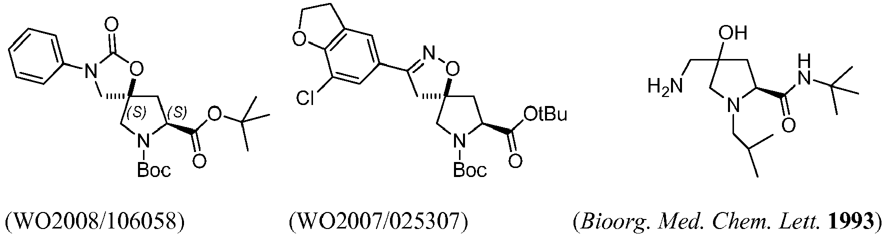

Spiroisoxazolines could be conceivably prepared from compounds of formula (II) by methods known to the person skilled in the art. These compounds have been prepared from exo-methylene Boc-proline derivatives in a 1,3-diploar cycloaddition with a nitrile oxide (WO2007/025307), and are used as precursors for pharmacetically active agents to inhibit serine protease activity.

Still other derivatives, conceivably prepared from compounds of formula (II) by methods known to the person skilled in the art, are precursors for HIV-1 protease inhibitors (Bioorg. Med. Chem. Lett. 1993, 3, 1485). These were obtained from ring opening of the precursor epoxide that was synthesized from *N*-*iso*-butyl 4-oxo proline *tert*-butylamide and sulfoxonium ylide as a mixture of diastereoisomers (2:1 in favor of the 4-(*S*) isomer). Both diasteroisomers were separated by chromatography.

Iodolactonization reactions involving a five-membered carbocyclic ring are known for example from J. Chem. Soc., Perkin Transactions 1, 1990, 6, 1535 and Tetrahedron Letters 1986, 27, 5467.

Surprisingly, despite the fact that halo-lactonization reactions are well known in chemistry, no precedence for the halo-lactonization involving an isolated five-membered heterocyclic ring such as the present pyrrolidine ring appears to be reported in the literature. The process of the present invention provides the stereospecific halo-lactonization of compounds of formula (I), which may readily available in enantiomerically pure form from the chiral pool, starting from the appropriate hydroxy-proline precursor. The halo-lactonization product of formula (II) is a versatile substrate for the synthesis of compounds of formula (III) and of novel proline derivatives with a quaternary stereocenter at C4. The present halo-lactonization process, thus, leads in a reduced number of steps, avoiding the use of toxic metal catalysts, to highly complex chiral building blocks. Products may be obtained in high enantiomeric purity making chromatographic separation of isomers unnecessary.

### Detailed description of the invention

1) In a first embodiment, the invention relates to a process for the synthesis of 1-(halo-methyl)-3-oxo-2-oxa-5-azabicyclo[2.2.1]heptane derivatives of the formula (II) 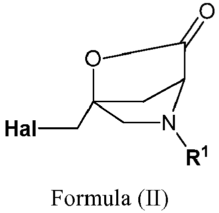 said process comprising the halo-lactonization of a compound of formula (I) 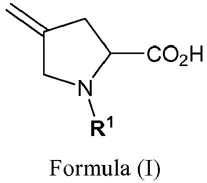 wherein
   **Hal** represents iodine or bromine (especially iodine); and
   **R¹** represents a nitrogen protecting group.
2) A second embodiment relates to the process according to embodiment 1), wherein said process is performed starting from the enantiomerically enriched L- or D- proline derivative of formula (Ia), respectively formula (Ib): 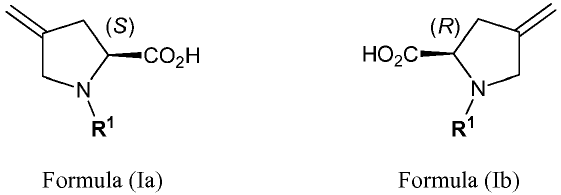 (especially starting from the essentially pure L-proline derivative of formula (Ia));
   thus, the process leads to the 1-(halo-methyl)-3-oxo-2-oxa-5-azabicyclo[2.2.1]heptane derivatives in enantiomerically enriched in the absolute configuration as depicted in formula (IIa), respectively, as depicted in formula (IIb): 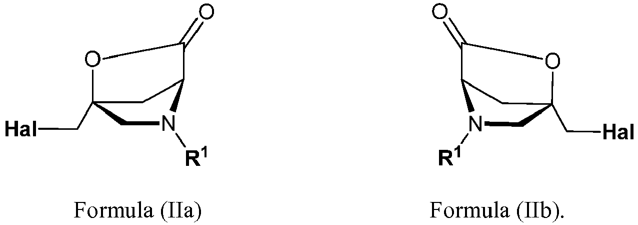
3) A further embodiment relates to the process of embodiments 1) or 2), wherein said halo-lactonization is performed in presence of a solvent.
   Solvents that may be used for the process of embodiment 1) are known in the art as being suitable for the well-known halo-lactonization reaction conditions. Such solvents may be aromatic solvents such as toluene, and anisol; ether solvents such as *tert.* butyl methyl ether (TBME), tetrahydrofurane (THF), dioxane, and 2-methyl-THF; chlorinated organic solvents such as dichloromethane, 1,2-dichloroethane, chlorobenzene, and 1,2-dichlorobenzene; esters such as ethyl acetate, isopropyl acetate, and n-butyl acetate; aprotic polar solvents such as DMF, dimethylacetamide, NMP, DMSO or acetonitrile; water; or mixtures of such solvents. Preferred solvents are the above-mentioned aprotic polar solvents such as especially DMF, dimethylacetamide, and NMP; or a mixture of water with such solvents. All solvents can be used as purchased without additional drying procedures.
4) A further embodiment relates to the process of embodiments 1) or 2), wherein said halo-lactonization is performed in presence of a solvent selected from DMF, water, or a mixture thereof.
5) A further embodiment relates to the process of any one of embodiments 1) to 4), wherein iodine or bromine; or an iodine- or bromine precursor (such as N-iodo-succinimide, N-bromosuccinimide, DBDMH (1,3-dibromo-5,5-dimethylhydantoin), or pyridine perbromide (pyridinium tribromide)) is used to perform the halo-lactonization.
6) A further embodiment relates to the process of embodiment 5), wherein about 1 to 10 equivalents, especially about 1 to 5 equivalents, of iodine, respectively bromine, respectively said iodine- or bromine precursor, with respect to the compound of Formula (I) are used.
7) A further embodiment relates to the process of embodiment 5), wherein about 3 equivalents, of iodine, respectively bromine, with respect to the compound of Formula (I) are used.
8) A further embodiment relates to the process of any one of embodiments 1) to 7), wherein said halo-lactonization is performed in presence of a base, or in absence of a base. In a preferred sub-embodiment, the process of any one of embodiments 1) to 7) is performed in presence of a base.
   Bases that are suitable for the halo-lactonization reaction are well known in the art and not particularly limited. Preferred are well known readily available and cheap bases; which in addition are commercially available implicating low cost of goods. Preferably, such base, when added to the reaction mixture, leads to a pH of about 7 to 14 (especially 8 to 10) in the reaction mixture. Preferred examples of such bases are aqueous base solutions, or aqueous buffer solutions; or mixtures thereof. In addition, under aprotic solvent reaction conditions for example alkali metal alcoholates may be used: N-iodoscuccinimide (NIS), potassium tert butoxide (KOtBu) or sodium ethoxide (NaOEt), DMF (tetrahedron 1979, 35, 2337 and Tetrahedron Latters 1990, 31, 5973).
   Most preferred bases are aq. base solutions. Examples of aqueous base solutions are alkali metal carbonate solutions, or an alkali metal hydrogen carbonate solutions, such as especially aq. Na₂CO₃, aq. NaHCO₃, aq. Li₂CO₃, aq. LiHCO₃, aq. K₂CO₃, aq. KHCO₃, aq. Cs₂CO₃, or aq. CsHCO₃; wherein said solutions may be saturated or diluted as appropriate.
   An aqueous buffer solution is notably sodium or potassium phosphate buffer (wherein said buffer may be diluted as appropriate (for example a 20 mM Na₃PO₄ buffer) adjusted to the appropriate pH; such as for example a pH of about 8-10) or other aqueous buffer systems known to the person skilled in the art.
   The above-mentioned aqueous base is usually present in large excess, especially in case a saturated solution is used, such aqueous base is present in amounts of about 1 to 15 vol, notably about 5 to 12 vol with respect to the compound of Formula (I).
   In the absence of a base, the halo-lactonization is reversible, and the thermodynamically most stable product is formed [using for example I₂, KI, acetonitrile (J. Am. Chem. Soc. 1978, 100, 3950)]
9) A further embodiment relates to the process of any one of embodiments 1) to 7), wherein said halo-lactonization is performed in presence of an aqueous alkali metal carbonate or hydrogen carbonate solution.
10) A further embodiment relates to the process of any one of embodiments 1) to 9), wherein said halo-lactonization is performed at a temperature of about -20°C to 50°C (especially at about 0°C to 40°C, notably at about room temperature.
11) A further embodiment relates to the process of any one of embodiments 1) to 10), wherein said compound of Formula (II) is obtained by liquid-liquid separation and / or solid-liquid separation.
12) A further aspect of the present invention relates to a process according to any one of embodiments 1) to 11), wherein the compound of the formula (II) is further transformed to a compound of the formula (III): 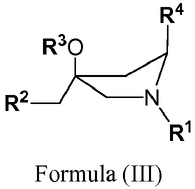 wherein
   **R¹** represents a nitrogen protecting group;
   **R²** represents hydrogen, iodine, bromine, hydroxy, alkoxy, or an optionally substituted amino group;
   **R³** represents hydrogen;
   **R⁴** represents -CH₂-OH; or COO-**R⁴¹**, wherein **R⁴¹** represents hydrogen, C₁₋₈-alkyl, C₁₋₃-fluroroalkyl, or optionally substituted aryl-C₁-₆-alkyl (especially hydrogen, C₁-₄-alkyl, or optionally substituted benzyl).
   It is understood that in case compounds of formula (II) in enantiomerically enriched form (i.e. compounds of formula (IIa) / (IIb) are used in the process of embodiment 12), the corresponding enantiomerically enriched compound of formula (III) will result.
13) A further embodiment relates to the process of embodiment 12), wherein said transformation of the compound of formula (II) to the compound of formula (III) comprises a ring opening reaction of the lactone ring of the compounds of formula (II).
14) A further embodiment relates to the process of embodiment 13), wherein said ring opening of the lactone ring of the compounds of formula (II) is achieved under acid catalysis; or said ring opening of the lactone ring of the compounds of formula (II) is achieved by nucleophilic attack under neutral or basic conditions.
   Acid catalysts suitable for the ring opening of a lactone (i.e. acid catalysts capable of activating the carbonyl group part of the lactone ring for a subsequent nucleophilic attack by a compound of formula HO-R⁴¹) are well-known in the art and encompass Lewis acids and Bronsted acids. A particularly suitable example is H₂SO₄.
   Reagents capable of a nucleophilic attack on the lactone ring (i.e. on the carbonyl group part of the lactone ring) are for example well-known hydride reagents (such as NaBH₄ or LiBH₄); or a compound of the formula HO-R⁴¹, a salt thereof (wherein such salt may be formed *in situ*); or a mixture of a compound of the formula HO-R⁴¹ and a salt thereof.
15) A further embodiment relates to the process of any one of embodiments 12) to 14), wherein said process is performed in presence of one or more solvents.
   Solvents that are suitable for the well-known ring opening of lactones and that in consequence may be used for the process of embodiments 12) to 14) are known in the art.
   In some instances, the nucleophil used for the ring opening of the lactone ring may serve as a solvent, for example in case it is water or an alcohol; or said nucleophil is a salt of said solvent (e.g. an alkali metal alcoholate; an alkali metal hydroxide; or ammonium hydroxide) and the reaction performed in presence of a mixture / solution of said solvent and a suitable amount of said salt of the solvent.
   Chemically inert solvents that may be used (in some instances in addition to the nucleophil being itself a solvent as described above) are for example: aromatic solvents such as toluene; ether solvents such as *tert.* butyl methyl ether (TBME), tetrahydrofurane (THF), dioxane, and 2-methyl-THF; chlorinated organic solvents such as dichloromethane, 1,2-dichloroethane, chlorobenzene, and 1,2-dichlorobenzene; aprotic polar solvents such as DMF, dimethylacetamide, acetonitrile, NMP, or DMSO; water; or mixtures of such solvents. All solvents can be used as purchased without additional drying procedures.
   Preferably, the process of embodiments 12) to 14) is performed in
   ➢ an alcoholic solvent in case the nucleophil is an alkali metal alcoholate;
   ➢ in DMSO or THF in case the nucleophil is a hydride reagent such as an alkali metal borohydride;
   ➢ in an ether solvent (such as especially TBME) in case an acid catalyst is used in presence of an alcohol nucleophil (wherein said alcohol may serve as a co-solvent); or
   ➢ in a mixture of an ether solvent (such as especially THF) and water in case the nucleophil is an alkali metal hydroxide or ammonium hydroxide.
16) A further embodiment relates to the process of any one of embodiments 12) to 15), wherein the nucleophil itself serves as a solvent; especially such nucleophil is an alcohol or water; wherein it is understood that said nucleophil may be present in free form or, at least partly, in salt form, optionally in a mixture with one or more chemically inert solvent(s).
17) A further embodiment relates to the process of any one of embodiments 12) to 16), wherein at least about 5 to 10 equivalents, especially at least about 5 to 10 equivalents, of nucleophil, with respect to the compound of Formula (II) are used.
18) A further embodiment relates to the process of any one of embodiments 12) to 17), wherein said ring opening is performed in presence of an acidic catalyst.
19) A further embodiment relates to the process of embodiment 18), wherein said ring opening is performed in presence of an alcohol.
20) A further embodiment relates to the process of any one of embodiments 12) to 17), wherein said ring opening is performed under basic conditions.
21) A further embodiment relates to the process of any one of embodiments 20), wherein said ring opening is performed in presence of an aqueous alkali metal hydroxide, or in presence of aqueous ammonium hydroxide.
22) A further embodiment relates to the process of embodiments 12) to 15), or 20), wherein said ring opening is performed in presence of an alkali metal borohydride.
23) A further embodiment relates to the process of any one of embodiments 12) to 22), wherein said ring opening is performed at a temperature of about -20°C to 100°C (especially at about 0°C to 40°C, notably at about room temperature).
24) A further embodiment relates to the process of any one of embodiments 12) to 23), wherein said compound of Formula (III) is obtained by liquid-liquid separation and / or solid-liquid separation.
25) A further aspect of the present invention relates to novel compounds of the formula (II): 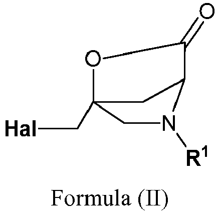 wherein
   **R¹** represents a nitrogen protecting group; and
   **Hal** represents bromine, or especially iodine;
   wherein in a sub-embodiment the compound of formula (II) is enantiomerically enriched in the absolute configuration as depicted in formula (IIa), respectively, as depicted in formula (IIb).
26) A further aspect of the present invention relates to novel compounds of the formula (III): 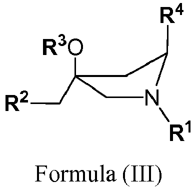 as defined for the process of embodiment 12); wherein said compound is:
   (2S,4S)-1-(tert-butoxycarbonyl)-4-hydroxy-4-(methoxymethyl)-pyrrolidine-2-carboxylic acid;
   (2S,4S)-1-(tert-butoxycarbonyl)-4-hydroxy-4-(hydroxymethyl)-pyrrolidine-2-carboxylic acid;
   (2S,4S)-4-hydroxy-4-(methoxymethyl)-1-tosylpyrrolidine-2-carboxylic acid;
   (2S,4R)-4-((benzylamino)methyl)-1-(tert-butoxycarbonyl)-4-hydroxypyrrolidine-2-carboxylic acid;
   (2S,4R)-4-(aminomethyl)-4-hydroxy-1-tosylpyrrolidine-2-carboxylic acid;
   (2S,4S)-methyl 4-hydroxy-4-(iodomethyl)-1-tosyl-pyrrolidine-2-carboxylate;
   (2S,4R)-1-tert-butyl-2-methyl-4-((1,3-dioxoisoindolin-2-yl)methyl)-4-hydroxypyrrolidine-1,2-dicarboxylate;
   (2S,4R)-methyl-4-((1,3-dioxoisoindolin-2-yl)methyl)-4-hydroxy-1-tosylpyrrolidine-2-carboxylate;
   (2S,4S)-benzyl-4-hydroxy-2-(hydroxymethyl)-4-methyl-pyrrolidine-1-carboxylate; or
   (2S,4S)-tert-butyl-4-hydroxy-2-(hydroxymethyl)-4-(iodomethyl)-pyrrolidine-1-carboxylate.
27) A further aspect of the present invention relates to the compound (5S,8S)-7-(tert-butoxycarbonyl)-2,2-dimethyl-1,3-dioxa-7-azaspiro[4.4]nonane-8-carboxylic acid.

The term "nitrogen protecting group" as used for the substituent **R¹** means any residue other than hydrogen which protects the nitrogen atom by forming a covalent bond to said nitrogen wherein said bond is not affected by the reaction conditions of the present halo-lactonization; and wherein preferably said residue itself is not chemically transformed under the conditions of the present halo-lactonization. The term includes residues which are not removed in subsequent steps (i.e. which may be synthetically useful for the further transformations of the compounds of formula (II) into the final target molecules thus incorporating such nitrogen protecting groups or derivatives thereof), thus residues other than generally recognized nitrogen protecting groups as listed for example in "Green's Protective Groups in Organic Synthesis", 4th edition, 2006, T.W. Greene, P.G.M. Wuts, Wiley-Interscience, 2006, which is incorporated by reference. Preferred are nitrogen protecting groups as defined herein-above which allow at least partly for a delocalization of the nitrogen lone pair (i.e. which may reduce the nitrogen basicity / nucleophilicity).

In a sub-embodiment, the term "nitrogen protecting group" as used for the substituent **R¹** represents optionally substituted aryl, optionally substituted heteroaryl; or especially a group forming together with the nitrogen of the pyrrolidine ring an amide, a carbamate, an urea, a sulfamate, a sulfonamide, or a sulfamide group. Notably, the term represents optionally substituted aryl; or, preferably, a group forming together with the nitrogen an amide, a carbamate, an urea, a sulfamate, a sulfonamide, or a sulfamide group.

Such groups forming together with the nitrogen of the pyrrolidine ring an amide, a carbamate, an urea, a sulfamate, a sulfonamide, or a sulfamide group may be selected from:
_{➢} **R¹¹-X**-CO- wherein
   ■ X represents a direct bond, oxygen, or N**R⁵** wherein **R⁵** represents C₁₋₈-alkyl, C₁-₃-fluroroalkyl, optionally substituted aryl, optionally substituted aryl-C₁₋₆-alkyl-, optionally substituted heteroaryl, optionally substituted heteroaryl-C₁-₆-alkyl-; and
   ■ **R¹¹** represents C₁-₈-alkyl, C₁-₃-fluroroalkyl, optionally substituted aryl, optionally substituted aryl-C₁-₆-alkyl-, optionally substituted heteroaryl, optionally substituted heteroaryl-C₁-₆-alkyl-, or
   ■ in case **X** represents N**R⁵**, **R¹¹** together with **R⁵** and the nitrogen to which both are attached may form a 4 to 8 membered ring optionally containing a further heteroatom selected from nitrogen and oxygen;
➢ **R¹²-Y**-SO₂- wherein
   ■ Y represents a direct bond, oxygen, or N**R⁶** wherein **R⁶** represents C₁-₈-alkyl, C₁-₃-fluroroalkyl, optionally substituted aryl, optionally substituted aryl-C₁-₆-alkyl-, optionally substituted heteroaryl, optionally substituted heteroaryl-C₁-₆-alkyl-;
   ■ **R¹²** represents C₁-₈-alkyl, C₁-₃-fluroroalkyl, optionally substituted aryl, optionally substituted aryl-C₁-₆-alkyl-, optionally substituted heteroaryl, optionally substituted heteroaryl-C₁-₆-alkyl-, or
   ■ in case **Y** represents N**R⁶**, **R¹²** together with **R⁶** and the nitrogen to which both are attached may form a 4- to 8-membered ring optionally containing a further heteroatom selected from nitrogen and oxygen.

In particular the term refers to the above groups forming together with the nitrogen an amide, a carbamate, or a sulfonamide group.

Examples of such "nitrogen protecting groups" as used for the substituent **R¹** are notably C₁-₈-alkoxy-carbonyl; C₁-₈-alkyl-carbonyl; C₁-₃-fluoroalkyl-carbonyl; optionally substituted aryloxy-sulfonyl, or C₁-₈-alkyloxy-sulfonyl (thus forming together with the nitrogen a sulfamate group); optionally substituted arylamino-sulfonyl, or C₁-₈-alkylamino-sulfonyl (thus forming together with the nitrogen a sulfamide group); and optionally substituted aryl-sulfonyl, or C₁-₈-alkylsulfonyl (thus forming together with the nitrogen a sulfonamide group). Particular examples are *tert*-butyloxycarbonyl, benzyloxycarbonyl, optionally substituted aryl-sulfonyl (such as especially toluene-sulfonyl), optionally substituted benzoyl (such as especially unsubstituted benzoyl), and optionally substituted phenyl (such as especially 2-nitro-phenyl). Preferred examples are *tert*-butyloxycarbonyl, benzyloxycarbonyl, optionally substituted aryl-sulfonyl (especially toluene-sulfonyl), and optionally substituted benzoyl (especially unsubstituted benzoyl),

The term "optionally substituted amino group" as used for the substituent **R²** refers to a residue comprising at least one nitrogen atom that is covalently bonded to the rest of the molecule (wherein it is understood that said nitrogen which is part of said optionally substituted amino group **R²,** before being covalently bonded to the rest of the molecule, had nucleophilic properties and a free valency). Such optionally substituted amino group **R²** notably is a group - N**R⁷R⁸**, wherein **R⁷** and **R⁸** independently represent hydrogen or C₁-₈-alkyl, or **R⁷** and **R⁸** together with the nitrogen to which they are attached form a 4- to 8-membered ring optionally containing a further heteroatom selected from optionally substituted nitrogen and oxygen, or **R⁷** and **R⁸** together with the nitrogen to which they are attached form an optionally substituted phthalimide ring.

In case a nitrogen atom is optionally substituted, the optional substituents are not particularly limited. Especially encompassed are substituents such as C₁₋₄-alkyl, C₃₋₆-cycloalkyl, C₁₋₃-fluoroalkyl, C₁₋₄-alkoxy-C₂₋₃-alkyl, hydroxy-C₂₋₃-alkyl, C₃₋₆-cycloalkoxy- C₁₋₄-alkyl, as well as "nitrogen protecting groups" forming together with the nitrogen an amide, a carbamate, an urea, a sulfamate, a sulfonamide, or a sulfamide group, as defined before.

The term "halogen" means fluorine, chlorine, or bromine, preferably fluorine or chlorine. As a sub-group of halogen, the term **Hal** refers to iodine or bromine, especially iodine.

The term "alkyl", used alone or in combination, refers to a saturated straight or branched chain alkyl group containing one to eight carbon atoms. The term "C_{x-y}-alkyl" (x and y each being an integer), refers to an alkyl group as defined before, containing x to y carbon atoms. For example a C₁₋₄-alkyl group contains from one to four carbon atoms. Examples of alkyl groups are methyl, ethyl, propyl, isopropyl, n-butyl, isobutyl, sec.-butyl and tert.-butyl. Preferred are methyl and ethyl. Most preferred is methyl.

The term "cycloalkyl", used alone or in combination, refers to a saturated cyclic alkyl group containing three to six carbon atoms. The term "C_{x-y}-cycloalkyl" (x and y each being an integer), refers to a cycloalkyl group as defined before containing x to y carbon atoms. For example a C₃₋₆-cycloalkyl group contains from three to six carbon atoms. Examples of cycloalkyl groups are cyclopropyl, cyclobutyl, cyclopentyl and cyclohexyl. Preferred is cyclopropyl.

The term "alkoxy", used alone or in combination, refers to an alkyl-O- group wherein the alkyl group refers to a saturated straight or branched chain alkyl group containing one to eight carbon atoms. The term "C_{x-y}-alkoxy" (x and y each being an integer) refers to an alkoxy group as defined before containing x to y carbon atoms. For example a C₁₋₄-alkoxy group means a group of the formula C₁₋₄-alkyl-O- in which the term "C₁₋₄-alkyl" has the previously given significance. Examples of alkoxy groups are methoxy, ethoxy, n-propoxy, isopropoxy, n-butoxy, isobutoxy, sec.-butoxy and tert.-butoxy. Preferred are ethoxy and methoxy.

The term "fluoroalkyl" refers to an alkyl group as defined before containing one to three carbon atoms in which one or more (and possibly all) hydrogen atoms have been replaced with fluorine. The term "C_{x-y}-fluoroalkyl" (x and y each being an integer) refers to a fluoroalkyl group as defined before containing x to y carbon atoms. For example a C₁₋₃-fluoroalkyl group contains from one to three carbon atoms in which one to seven hydrogen atoms have been replaced with fluorine. Representative examples of fluoroalkyl groups include trifluoromethyl, 2-fluoroethyl, 2,2-difluoroethyl and 2,2,2-trifluoroethyl. Preferred are C₁-fluoroalkyl groups such as trifluoromethyl.

The term "fluoroalkoxy" refers to an alkoxy group as defined before containing one to three carbon atoms in which one or more (and possibly all) hydrogen atoms have been replaced with fluorine. The term "C_{x-y}-fluoroalkoxy" (x and y each being an integer) refers to a fluoroalkoxy group as defined before containing x to y carbon atoms. For example a C₁₋₃-fluoroalkoxy group contains from one to three carbon atoms in which one to seven hydrogen atoms have been replaced with fluorine. Representative examples of fluoroalkoxy groups include trifluoromethoxy, difluoromethoxy, 2-fluoroethoxy, 2,2-difluoroethoxy and 2,2,2-trifluoroethoxy. Preferred are C₁-fluoroalkoxy groups such as trifluoromethoxy and difluoromethoxy.

The term "aryl" refers to a naphthyl or, preferably, to a phenyl group; wherein said group is unsubstituted or substituted as explicitly defined.

In case an aryl group is optionally substituted, the optional substituents are not particularly limited. Especially encompassed are aryl groups wherein the aryl group is unsubstituted, or mono-, di-, or tri-substituted; wherein the substituents are independently selected from C₁₋₄-alkyl, C₁₋₄-alkoxy, C₃₋₆-cycloalkyl, halogen, C₁₋₃-fluoroalkyl, C₁₋₃-fluoroalkoxy; hydroxy, C₁₋₄-alkoxy-C₁₋₃-alkyl, hydroxy-C₁₋₃-alkyl, C₁₋₄-alkoxy-C₂₋₃-alkoxy, hydroxy-C₂₋₃-alkoxy, carboxy, C₁₋₄-alkoxy-carbonyl, C₃₋₆-cycloalkoxy-carbonyl, nitro, and cyano.

The term "heteroaryl", if not explicitly stated otherwise, refers to a 5- to 10-membered monocyclic or bicyclic aromatic ring containing 1 to a maximum of 4 heteroatoms independently selected from oxygen, nitrogen and sulfur. Examples of such heteroaryl groups are 5-membered monocyclic heteroaryl groups such as furanyl, oxazolyl, isoxazolyl, oxadiazolyl, thienyl, thiazolyl, isothiazolyl, thiadiazolyl, pyrrolyl, imidazolyl, pyrazolyl, triazolyl and tetrazolyl; 6-membered monocyclic heteroaryl such as pyridyl, pyrimidyl, pyridazinyl, and pyrazinyl; and 8- to 10-membered bicyclic heteroaryl such as indolyl, isoindolyl, benzofuranyl, isobenzofuranyl, benzothiophenyl, indazolyl, benzimidazolyl, benzoxazolyl (or benzooxazolyl), benzisoxazolyl, benzothiazolyl, benzoisothiazolyl, benzotriazolyl, benzoxadiazolyl, benzothiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidyl, imidazo[1,2-a]pyridyl, 1H-pyrrolo[3,2-b]pyridyl, 1H-pyrrolo[2,3-b]pyridyl, pyrrolo[3,2-d]pyrimidinyl, pyrrolo[2,3-d]pyrimidinyl, 4H-furo[3,2-b]pyrrolyl, pyrrolo[2,1-b]thiazolyl, and imidazo[2,1-b]thiazolyl. The above-mentioned heteroaryl groups are unsubstituted or substituted as explicitly defined.

In case a heteroaryl group is optionally substituted, the optional substituents are not particularly limited. Especially encompassed are heteroaryl groups which are unsubstituted, or mono-, di-, or tri-substituted; wherein the substituents are independently selected from C₁₋₄-alkyl, C₁₋₄-alkoxy, C₃₋₆-cycloalkyl, halogen, C₁₋₃-fluoroalkyl, C₁₋₃-fluoroalkoxy; hydroxy, C₁₋₄-alkoxy-C₁₋₃-alkyl, hydroxy-C₁₋₃-alkyl, C₁₋₄-alkoxy-C₂₋₃-alkoxy, hydroxy-C₂₋₃-alkoxy, carboxy, C₁₋₄-alkoxy-carbonyl, C₃₋₆-cycloalkoxy-carbonyl, nitro, and cyano.

In case more than one group are combined in one generic group (for example: aryl-C₁₋₆-alkyl), it is understood that such group is to be read from left to right (i.e. in the above example: the aryl-C₁₋₆-alkyl is attached to the rest of the molecule at the C₁₋₆-alkyl group).

The term "solid-liquid separation" refers to routine solid-liquid separation techniques well known to a skilled person (see for example Perry's Chemical Engineers' Handbook, 7th edition, Perry, R.H.; Green, D. W. McGraw-Hill 1997). In particular, the term includes techniques such as filtration, centrifugation, and gravity sedimentation; especially filtration.

The term "liquid-liquid extraction" refers to routine liquid-liquid extraction or washing techniques well known to a skilled person (see for example Perry's Chemical Engineers' Handbook, 7th edition, Perry, R.H.; Green, D. W. McGraw-Hill 1997). In particular the term includes washing or extraction techniques using settlers, cyclones, centrifuges, mixer-settler, all kinds of continuous contact equipment; distillation: batch and continuous distillation; and supercritical fluid separation techniques.

Unless used regarding temperatures, the term "about" placed before a numerical value "X" refers in the current application to an interval extending from X minus 10% of X to X plus 10% of X, and preferably to an interval extending from X minus 5% of X to X plus 5% of X. In case the term about is placed before a range, the respective interval is to be applied to both values of the range. In the particular case of temperatures, the term "about" placed before a temperature "Y" refers in the current application to an interval extending from the temperature Y minus 10 °C to Y plus 10 °C, and preferably to an interval extending from Y minus 5 °C to Y plus 5 °C.

Whenever the word "between" or "to" is used to describe a numerical range, it is to be understood that the end points of the indicated range are explicitly included in the range. For example: if a temperature range is described to be between 40°C and 80°C (or 40°C to 80°C), this means that the end points 40°C and 80°C are included in the range; or if a variable is defined as being an integer between 1 and 4 (or 1 to 4), this means that the variable is the integer 1, 2, 3, or 4.

The expression % w/w refers to a percentage by weight compared to the total weight of the composition considered. Likewise, the expression v/v refers to a ratio by volume the two components considered. Likewise, the expression % a/a refers to the purity with respect to area under the curve (i.e. integral) in a chromatogram, preferably measuring the UV absorption. The expression "vol" signifies volumes (in L, e.g. of solvent) per weight (in kg, e.g. of reactant). For example 7 vol signifies 7 liters (of solvent) per kg (of reactant).

The term "enriched", for example when used in the context of enantiomers or diastereoisomers is understood in the context of the present invention to mean especially that the respective enantiomer / diastereoisomer is present in a ratio (mutatis mutandis: purity) as explicitly specified; usually in a ratio of at least 60:40, especially of at least 70:30, and notably of at least 90:10 (mutatis mutandis: purity of 60% / 70% / 90%) with respect to the respective other enantiomer / diastereoisomer. Preferably the term refers to the respective essentially pure enantiomer / diastereoisomer.

The term "essentially", for example when used in a term such as "essentially pure" is understood in the context of the present invention to mean especially that the respective stereoisomer / composition / compound etc. consists in an amount of at least 90, especially of at least 95, and notably of at least 99 per cent by weight of the respective pure stereoisomer / composition / compound etc..

### Examples

The following examples illustrate the invention but do not at all limit the scope thereof.

All temperatures are stated in °C. Compounds are characterized by ¹H-NMR (400MHz) or ¹³C-NMR (100MHz) (Brucker; chemical shifts are given in ppm relative to the solvent used; multiplicities: s = singlet, d = doublet, t = triplet; p = pentuplet, hex = hexet, hept = heptet, m = multiplet, br = broad, coupling constants are given in Hz); by LC-MS (Finnigan Navigator with HP 1100 Binary Pump and DAD, column: 4.6x50 mm, Zorbax SB-AQ, 3.5 m, 120A, gradient: 5-95% acetonitrile in water/NH₃ 13 mmol/L, 1 min, with NH₄(COO) 10 mmol/L, flow: 4.5 ml/min) or column: 4.6x50 mm, Zorbax SB-AQ, 3.5 m, 120A, gradient: 5-95% acetonitrile in water, 1 min, with 0.04% TFA, flow: 4.5 ml/min), t_{R} is given in min; by TLC (TLC-plates from Merck, Silica gel 60 F254); or by melting point. Compounds are purified by flash chromatography, crystallization or preparative HPLC (Pump: Gilson 333/334; Column: Waters Atlantis T3 30x75 mm 10 µm, Waters XBridge C18, 30x75 mm, 10 µm, Waters C18, 50x150 mm, 10 µm; flow rate: 75 mL/min for 30x75 mm or 150 ml/min for 50x150 mm; mobile phase: A: water + 0.5% formic acid or water + 0.5% ammonium hydroxid 25%, B: acetonitrile).

### Abbreviations

- aq.: aqueous
- AcOEt: ethyl acetate
- atm: atmosphere
- Bn: benzyl
- Boc: *tert*.-butyloxycarbonyl
- CAM:
- CBz: benzyloxycarbonyl
- DCM: dichloromethane
- DMF: dimethylformamide
- DMSO: dimethylsulfoxide
- ee: enantiomeric excess
- equ.: equivalent(s)
- e.r.: enantiomeric ratio
- ET: external temperature
- EtOAc: ethyl acetate
- IPC: In Process Control
- iPrOAc: isopropyl acetate
- GC-MS: Gas Chromatography - Mass Spectroscopy
- h: hour(s)
- HV: high vacuum conditions
- IPC: In process control
- LC-MS: Liquid Chromatography - Mass Spectrometry
- m.p.: melting point
- min: minutes
- org.: organic
- rt: room temperature
- sat.: saturated
- soln.: solution
- t_{R}: retention time
- TBME: *tert*.-butyl methyl ether
- TFA: trifluoroacetic acid
- THF: tetrahydrofuran
- TLC: thin layer chromatography
- Ts: tosyl (para-toluene-sulfonyl)
- % w/w: Mass % (NMR assay)
- % a/a: Area % (purity by area%)

### Iodo - lactonizations

### Example 1: (1S,4S)-tert-butyl 1-(iodomethyl)-3-oxo-2-oxa-5-azabicyclo[2.2.1]heptane-5-carboxylate

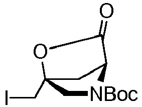

A solution of N-Boc-4-methylene-L-proline (50 g, 0.22 mol) in DMF (50 mL, 7 vol) was added to sat. aq. NaHCO₃ soln. (600 mL, 12 vol) at 20-25 °C during 20 min (evolution of gas, pH at the end of addition = 8-9). The resulting solution was treated with a solution of I₂ (167.5 g, 0.66 mol, 3.0 equ) in DMF (350 mL, 7 vol) and water (100 ml, 2 vol) at 20-25 °C during 30 min (evolution of gas). The reaction was further stirred at 20-25 °C for 1.5 h at 20-25 °C.
The reaction was diluted with AcOEt (700 mL), water (200 mL) and aq. soln. of NaHSO₃ 40% (140 mL) during 10 min at 20-25 °C (strongly foaming). The layers were separated. The aq. layer was extracted 1x with AcOEt (250 mL). The combined org. layers were washed 1x with sat. aq. NaHCO₃ (250 mL) and 1x with water (250 mL). The org. layer was concentrated under reduced pressure to give 71 g (91%) of iodo lactone as a yellow-white solid. MP = 131-133 °C. LC-MS: t_{R} = 1.34 min. ¹H-NMR (D₆ DMSO, 400MHz) δ 4.61 (s, 1 H), 3.80-3.70 (m, 2 H), 3.62-3.53 (m, 1 H), 3.34-3.27 (m, 1 H), 2.2.23-2.14 (m, 2 H), 1.43-1.34 (m, 9 H).

The absolute configuration of the obtained title compound was confirmed by X-ray.

### Example 2: (1S,4S)-tert-butyl 1-(bromomethyl)-3-oxo-2-oxa-5-azabicyclo[2.2.1]heptane-5-carboxylate:

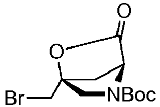

A solution of N-Boc-4-Methylene-L-proline (7.1 g, 0.03 mol) in DMF (15 mL, 2.1 vol) was added to sat. aq. NaHCO₃ soln. (50 mL, 7 vol) at 20-25 °C during 7 min (evolution of gas). The resulting solution was treated with a solution of Br₂ (10.0 g, 0.06 mol, 2.0 equ) in DMF (20 mL, 3 vol) at 20-25 °C during 15 min. The reaction was further stirred at 20-25 °C for 2 h at 20-25 °C. The reaction was diluted with AcOEt (50 mL). Then it was quenched by addition of aq. soln. of NaHSO₃ 40% (12 mL) during 5 min at 20-25 °C (strongly foaming). The layers were separated. The aq. layer was extracted 1x with AcOEt (35 mL). The combined org. layers were washed with sat. aq. 10% NaHSO₃ (1x 35 mL), with sat. aq. NaHCO₃ soln. (1x 35 mL) and with water (1x 35 mL). The org. layer was concentrated under reduced pressure to give crude bromo lactone as a yellow oil (3.7 g). The crude product was further purified by a plug filtration over silica gel (55 g) using heptane/ethyl acetate 4:1 (1.7 L) to give 2.37 and and a column chromatography (Biotage Snap 25 g; gradient = heptane (100 ml) then heptane/ethylacetate 0-30% (700 mL) then heptante/ethylacetate 7:3 (200 ml)) to give 1.11 g (12%) of pure bromo lactone as a white solid. MP = 147-149 °C. LC-MS: t_{R} = 1.34 min. ¹H-NMR (CDCl₃, ppm, 400MHz) δ 4.66 (s, broad, 1H), 3.75 (s, 2 H), 3.67-3.61 (m, 1 H), 3.55-3.4 (m, 1 H), 2.23-2.10 (m, 2 H), 1.49 (s, 9 H).

### Example 3: (1S,4S)-1-(iodomethyl)-5-tosyl-2-oxa-5-azabicyclo[2.2.1]heptan-3-one

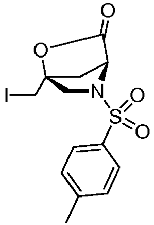

### Step 1: (S)-methyl 4-methylene-1-tosylpyrrolidine-2-carboxylate

A suspension of (*S*)-methyl 4-methylenepyrrolidine-2-carboxylate hydrochloride (4.0 g, 22.5 mmol) in DCM (32 mL) was treated with triethylamine (7.8 mL, 2.5 eq.) and p-toluenesulfonylchloride (4.8 g, 1.1 eq.) at 25-43 °C. After 1.5 h stirring at 20-25 °C an IPC (TLC) showed complete consumption of starting material. After standing over night the reaction mixture was extracted with aq. 1 N HCl (2x 20 mL). The organic layer was evaporated at 50 °C under reduced pressure to give the crude ester (5.9 g) as a brown oil. It was dissolved in THF (17 mL) and with aq. 1 N NaOH (15 mL) at 20-25 °C. The reaction mixture was stirred at 20-25 °C. After 30 min an IPC (LC-MS) showed >99% conversion to acid. The reaction mixture was extracted with TBME (30 mL) and was acidified with aq. 32% HCl (3 mL; resulting pH was < 1). The mixture was extracted with DCM (30 mL). The organic layer was evaporated to give 6.3 g of crude acid. The crude product was further purified by flash chromatography (silica gel; ethyl acetate/methanol/acetic acid 400:10:1) to give 3.5 g (62%) of acid as a brown oil. The purity was >99% a/a. LC-MS: t_{R} = 0.74 min, [M+1]⁺ = 282. ¹H-NMR (CDCl₃, 400MHz) δ 12.89 (s, broad, 1 H), 7.73 (d, *J* = 8.2 Hz, 2 H), 7.43 (d, *J* = 8.2 Hz, 2 H), 4.98-4.93 (m, 2 H), 4.33 (dd, *J*₁ = 3.4 Hz, *J*₂ = 9.1 Hz, 1 H), 3.98-3.82 (m, 2 H), 2.55-2.48 (m, 2 H), 2.40 (s, 3 H).

*Step* 2: Sat. aq. NaHCO₃ (55 mL) was added to a solution of (*S*)-methyl 4-methylene-1-tosylpyrrolidine-2-carboxylate (4.8 g, 16.9 mmol) in DMF (12.5 mL). The resulting mixture was treated with a solution of iodine (12.9 g, 3.0 eq.) in DMF (22.5 ml). An IPC (LC-MS) showed complete conversion to product after 15 min at 20-30 min. The reaction was diluted with AcOEt (50 mL), aq. 20% NaHSO₃ (17 mL), water (50 mL) and DCM (100 mL). The layers were separated. The aq. layer was extracted with DCM (1x 100 mL). The organic layers were each washed with sat. aq. NaHCO₃ soln. (100 mL) and water (3x 100 mL). Then they were combined and evaporated under reduced pressure at 50 °C. After evaporating the solvents 5.6 g of crude iodolactone was obtained. It was suspended in AcOEt (25 mL). The mixture was heated to reflux. After cooling down to 0-5 °C 2.8 g of crystalline iodo lactone were isolated. The mother liquor was evaporated and the residue was subjected to a second crystallization from AcOEt to give a second crop of 0.7 g acid. The combined yield was 3.5 g (51%) as a off white solid. MP = 165-166 °C. LC-MS: t_{R} = 0.86 min. ¹H-NMR (D₆ DMSO, 400MHz) δ 7.72 (d, *J* = 8.3 Hz, 2 H), 7.46 (d, *J* = 8.2 Hz, 2 H), 4.68 (s, 1 H), 3.75-3.55 (m, 3 H), 3.26 (dd, *J*₁ = 1.1 Hz, *J*₂ = 9.9 Hz, 1 H), 2.42 (s, 3 H), 2.17 (d, *J* = 11.0 Hz, 1 H), 1.93 (dd, *J*₁ = 1.7 Hz, *J*₂ = 10.9 Hz, 1 H). ¹³C-NMR (D₆ DMSO, 100 MHz) δ 170 (C); 145 (C), 134 (C), 130 (CH), 128 (CH), 89 (C), 62 (CH), 54 (CH₂), 42 (CH₂), 22 (CH₃), 2 (CH₂).

### Example 4: (1S,4S)-benzyl 1-(iodomethyl)-3-oxo-2-oxa-5-azabicyclo[2.2.1]heptane-5-carboxylate

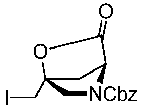

### Step 1: (S)-1-((benzyloxy)carbonyl)-4-methylenepyrrolidine-2-carboxylic acid:

(S)-methyl 4-methylenepyrrolidine-2-carboxylate hydrochloride (4.0 g, 23 mmol) was dissolved in THF/water 1:1 (48 mL). The solution was cooled to 0-5°C. After treatment with NaHCO₃ (8.5 g, 4.5 eq) the solution had pH 8. Z-chloride (3.9 mL, 1.2 eq) was added during 5 min at 0-5 °C. After 1 h stirring at 20-25 °C the reaction was diluted with DCM (50 mL). The layers were separated and the aq. layer was extracted with dichloromethane (1x 50 mL). The combined organic layers were washed with water (50 mL) and evaporated in vacuo at 50 °C to give 6 g of the crude ester. The crude methylester was dissolved in THF (12 mL). The solution was diluted with aq. 1 N NaOH (36 mL, 1.5 eq) at 20-25 °C. The resulting emulsion was stirred at 20-25 °C during 1 h. An IPC (LC-MS) showed complete conversion to free acid. The reaction mixture was diluted with water (100 mL) and the basic mixture was washed with DCM (2x 100 mL). The aqueous layer was acidified and extracted with DCM (3x 70 mL). The combined organic layers were evaporated to give 5.0 g of the free acid as a brown oil. LC-MS: t_{R} = 0.74 min, [M+1]⁺ = 262. ¹H-NMR (D₆ DMSO, 400MHz) δ 12.82 (s, broad, 1 H), 7.42-7.24 (m, 5 H), 5.15-5.00 (m, 4 H), 4.49-4.36 (m, 1 H), 4.13-3.95 (m, 2 H), 3.09-2.97 (m, 1 H), 2.61-2.44 (m, 1H).

*Step 2:* Sat. aq. NaHCO₃ solution (55 mL) was added to a solution of (S)-1-((benzyloxy)carbonyl)-4-methylenepyrrolidine-2-carboxylic acid (4.95 g,. 18.9 mmol) in DMF (15.5 mL) at 20-25 °C. After stirring during 10-15 min a solution of iodine (14.4 g, 3 eq) in DMF (22.5 ml) was added during 30 min at 25-35 °C. After completion of addition an IPC showed complete consumption of starting material. The reaction was diluted with AcOEt (50 mL) and aq. 40% NaHSO₃ (9 mL) in water (100 mL). After separation of layers the aqueous layer was extracted with ethyl acetate (2x 100 mL). Each organic layer was washed with sat aq. NaHCO₃ (2x 100 mL) and with water (100 mL). The organic layers were combined and evaporated to give the crude product as an oil still containing DMF. The oil was re-dissolved in iPrOAc (100 mL). The solution was washed with water (3x 100 mL). The aqeous layers were reextracted with iPrOAc (100 mL). The combined organic layers were evaporated to give 6.45 g of crude iodolactone. No further purification was performed. LC-MS: t_{R} = 0.86 min, [M+1]⁺ = 388. ¹H-NMR (D₆ DMSO, 400MHz) δ 7.41-7.34 (m, 5 H), 5.16-5.1 (m, 2 H), 4.75 (s, 1 H), 3.84-3.67 (m, 3 H), 3.39-3.32 (m, 1 H), 2.34-2.21 (m, 1 H). ¹³C-NMR (D₆ DMSO, 100 MHz) δ 171 (C), 163 (C), 154 (C), 137 (C), 129 (CH), 129 (CH), 128 (CH), 89 (C), 67 (CH₂), 60 (CH), 54 (CH₂), 43 (CH₂), 2 (CH₂)

### Example 5: (1S,4S)-5-benzoyl-1-(iodomethyl)-2-oxa-5-azabicyclo[2.2.1]heptan-3-one

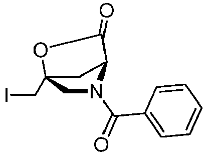

### Step 1: (S)-1-benzoyl 4-methylenepyrrolidine-2-carboxylic acid

Benzoic acid (3.6 g, 1.05 eq.) was dissolved in water (15 mL). The pH of the solution was adjusted to approx. 4 by addition of aq. 1 M NaOH (1 mL). Then 1-hydroxy-benzotriazole (4.6 g, 1.2 eq.) was added and the pH was again adjusted with aq. 32% NaOH (2 mL) to 4-5. Then a solution of (*S*)-methyl 4-methylenepyrrolidine-2-carboxylate hydrochloride (5 g, 28.1 mmol) in THF (25 ml) was added at 20-25 °C. The pH was readjusted from 1 to 4-5 by addition of aq. 32% NaOH (2 mL). The reaction was treated with a solution of 1-(3-dimethylaminopropyl)-3-ethylcarbodiimide HCl salt (6.5 g, 1.2 eq) in water (15 mL) at 20 - 33 °C during 10 min. After completion of addition the pH was adjusted from 3 to 5 with aq. 32% NaOH (1 mL). After 1 h 40 min at 20-25 °C an IPC (LC-MS and TLC (heptane/AcOEt 1:1; KMnO₄)) showed complete conversion to the amide. The reaction was diluted with DCM (20 mL) and sat. aq. NaHCO₃ solution (20 mL). The layers were separated, the organic layer was washed with aq. 10% citric acid (20 mL) and water (20 mL). Evaporation of all solvents gave 5.5 g of crude amide which was taken into the next step. Crude amide (5.5 g) was dissolved in THF (16 mL). The solution was treated with aq. 1 M NaOH (32 mL) at 20-25 °C was stirred during 1 h at 20-25 °C. An IPC (LC-MS) revealed complete hydrolysis of the ester. The reaction was extracted with TBME (25 mL). The aqueous layer was set to pH 1 and extracted DCM (2x 25 mL). The combined organic layers were evaporated to give 5.1 g of crude acid as a brown oil. Further purification was achieved by flash chromatography (150 g of silica gel; AcOEt/heptane 3:1, 2.1 L) to give 2.9 g (56%) of pure acid as a white solid. LC-MS: t_{R} = 0.62 min. ¹H-NMR (D₆ DMSO, 400MHz) δ 12.8 (s, broad, 1 H), 7.56-7.36 (m, 5 H), 5.13-4.95 (m, 2 H), 4.7-3.92 (m, several rotamers, 3 H), 3.14-2.95 (m, 1 H), 2.62 (d, *J* = 15.9 Hz, 1 H).

*Step 2:* Sat. aq. NaHCO₃ (32 mL) was placed into a flask and a solution of 2.9 g (12.4 mmol) of (S)-1-benzoyl-4-methylenepyrrolidine-2-carboxylic acid in DMF (7 mL) was added at 20-30 °C. The resulting reaction mixture was treated with a solution of iodine (30.6 mmol, 3.0 eq) in DMF (13 mL) at 20-30 °C. After stirring 1 h at 20-25 °C an IPC (LC-MS) showed > 99% conversion to iodolactone. The reaction was diluted with AcOEt (29 mL) and with aq. 40% NaHSO₃ (6 ml, 2.5 eq). The layers were separated and the aq. layer was extracted with DCM (100 mL). The organic layers were washed with water (100 mL). Evaporation of the combined organic layers gave 2.4 g (51%) of the iodolactone as a white solid. LC-MS: t_{R} = 0.75 min, [M+1]⁺ = 358. ¹H-NMR (D₆ DMSO, 400MHz) δ 7.64-7.48 (m, 5 H), 5.21-4.61 (m, rotamers, 1 H), 4.0-3.34 (m, broad, 4 H), 2.35-2.27 (m, 2 H). ¹³C-NMR (D₆ DMSO, 100 MHz) δ 135 (C), 131 (C), 129 (CH), 128, (CH), 89 (C), 63 (CH₂), 35 (CH), 31 (CH), 21 (CH); 2 (CH₂).

### Example 6: (1S,4S)-1-(iodomethyl)-5-(2-nitrophenyl)-2-oxa-5-azabicyclo[2.2.1]heptan-3-one

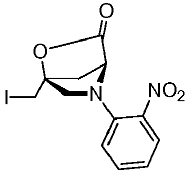

### Step 1: (S)-4-methylene-1-(2-nitrophenyl)pyrrolidine-2-carboxylic acid

(*S*)-methyl 4-methylenepyrrolidine-2-carboxylate hydrochloride (1.0 g, 5.6 mmol) in DMF (4 mL) was treated with potassium carbonate (1.63 g, 2.1 eq) at 20-25 °C. Then 1-fluoro-2-nitrobenzene (0.63 mL, 1.05 eq) was added dropwise at 0-5 °C. After 1 h the ice bath was removed. An IPC showed no reaction. Then the reaction was stirred over night at 20-25 °C. An IPC showed formation of both free acid and methylester of the product. After further 3 days at 20-25 ° and 1 day at 60 °C the reaction mixture was diluted with DCM (20 mL) and aq. 1 N NaOH (50 mL). It was stirred 1 h at 20-30 °C. The layers were separated and the aq. layer was acidified and extracted with DCM (50 mL). The organic layer was evaporated to give 620 mg crude product which was further purified by preparative HPLC. Purification gave 340 mg (24%) of the free acid as an orange oil. LC-MS: t_{R} = 0.77 min, [M+1]⁺ = 249. ¹H-NMR (D₆ DMSO, 400MHz) δ 12.93 (s, broad, 1 H), 7.71 (dd, *J*₁ = 1.4 Hz, *J*₂ = 8.1 Hz, 1 H), 7.50 (m, 1 H), 6.99 (d, *J* = 8.6 Hz, 1 H), 6.89 (t, *J* = 7.4 Hz, 1 H), 5.06 (d, *J* = 1.7 Hz, 2 H), 4.51 (dd, *J*₁ = 3.9 Hz, *J*₂ = 8.6 Hz, 1 H), 3.92 (m, 1 H), 3.78 (m, 1 H), 3.06 (m, 1 H), 2.72 (dd, *J*₁ = 1.8 Hz, *J*₂ = 15.7 Hz, 1 H). ¹³C-NMR (D₆ DMSO, 100 MHz) δ 173 (C), 144 (C), 141 (C), 139 (C), 134 (CH), 126 (CH), 118 (CH), 108 (CH₂), 62 (CH), 54 (CH₂), 36 (CH₂).

*Step 2:* A solution of (S)-4-methylene-1-(2-nitrophenyl)pyrrolidine-2-carboxylic acid (200 mg, 0.8 mmol) in DMF (0.5 mL) was added to sat. aq. NaHCO₃ (2.2 mL) at 20-25 °C. After the gas evolution ceased a solution of iodine (813 mg, 3 eq) in DMF (0.9 mL) was added. Another amount of DMF (3 mL) was required to enable stirring. After 2 h at 20-25 °C an IPC (LC-MS) showed complete conversion to lactone. The reaction was diluted with AcOEt (2 mL) and was carefully treated with aq. 40% NaHSO₃ (0.4 mL, 2.8 eq). Strong foam formation was observed. The reaction was further diluted with DCM (50 mL) and water (30 mL). The pH was adjusted to 10. The layers were separated. The aq. layer was extracted with DCM (2x 50 mL). All organic layers were washed with water (1x 50 mL) the combined organic layer was evaporated to give 260 mg (96%) of the iodolactone as a yellow solid. MP = 141 - 143 °C. LC-MS: t_{R} = 0.87 min, [M+1]⁺ = 375. ¹H-NMR (D₆ DMSO, 400MHz) δ 7.83 (m, 1 H), 7.59 (m, 1 H), 7.30 (d, *J* = 8.1 Hz, 1 H), 7.04 (m, 1 H), 5.07 (s, 1 H), 3.87-3.75 (m, 3 H), 2.57 (d, *J* = 9.8 Hz, 1 H), 2.35 (m, 2 H). ¹³C-NMR (D₆ DMSO, 100 MHz) δ 170 (C), 141 (C), 139 (C), 134 (CH), 127 (C), 120 (CH), 119 (CH), 87 (C), 61 (CH), 59 (CH₂), 43 (CH₂), 2 (CH₂).

### Derivatizations

### Example 7: (2S,4S)-1-tert-butyl 2-methyl 4-hydroxy-4-(iodomethyl)pyrrolidine-1,2-dicarboxylate

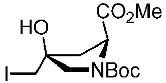

(1S,4S)-tert-butyl 1-(iodomethyl)-3-oxo-2-oxa-5-azabicyclo[2.2.1]heptane-5-carboxylate (Example 1; 5.3 g, 15 mmol) were suspended in TBME (20 ml) and MeOH (10 ml). To the resulting suspension H₂SO₄ conc. (0.1 ml) was added at 20-25 °C. The reaction was stirred at 20-25 °C during 3 h and was turned thereby into a turbid solution. The reaction was quenched by addition of sat. aq. NaHCO₃ (10 ml). Water (10 ml), TBME (20 ml). The layers were separated. The org. layer was washed 1x with water (10 ml). All solvents were removed to give 5.3 g of crude ester. Further purification was performed by column chromatography: Silica gel 60, EA:Heptane 1:3 gave 4.06 g (70%) of the ester as a purple oil that solidified slowly. TLC: Heptane/EtOAc 1:1, KMnO₄ or CAM stain. R_{f}(starting material) approx 0.7); R_{f}(product) approx 0.6. MP = 92 93 °C. LC-MS: t_{R} = 0.76 min, [M+1]⁺ = 386. ¹H-NMR (D₆ DMSO, ppm, 400MHz) δ 5.30 (d, *J* = 17.2 Hz, 1 H), 4.40-4.26 (m, 1 H), 3.66-3.61 (m, 2 H), 3.48-3.32 (m, 5 H), 2.39-2.30 (m, 1 H), 2.08-2.05 (m, 1 H), 1.42-1.28 (m, 9 H).

### Example 8: (2S,4S)-1-tert-butyl 2-methyl 4-hydroxy-4-(methoxymethyl)pyrrolidine-1,2-dicarboxylate

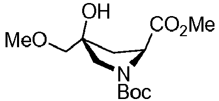

### Method A

(2S,4S)-1-tert-butyl 2-methyl 4-hydroxy-4-(iodomethyl)pyrrolidine-1,2-dicarboxylate (Example 1; 14 g, 36 mmol) were dissolved in methanol (70 mL). Then sodium methoxide 25% in methanol (17 mL, 2.0 eq.) was added at 20-25 °C. After 3 h stirring at 20-25 °C another amount of sodium methoxide 25% in methanol (8.3 mL, 1 eq.) was added. The reaction was stirred over night at 20-25 °C. Then it was poured onto 20% aq. citric acid at 20-25 °C.

The resulting pH was 5. The mixture was extracted with DCM (2x 70 mL). The organic layer was evaporated to dryness to give 9.7 g (93%) of the methoxylated product as a yellow oil.

### Method B

(1S,4S)-tert-butyl 1-(iodomethyl)-3-oxo-2-oxa-5-azabicyclo[2.2.1]heptane-5-carboxylate (Example 1; 1.0 g, 2.8 mmol) was dissolved in methanol (6 mL). The solution was treated with sodium methoxide 25% in methanol (2 mL, 8.5 mmol) at 20-25 °C. After 3 h at 20-25 °C a TLC showed only product. The reaction was quenched with aq. 20% citric acid (6 mL) at 20-25 °C. The reaction mixture was extracted with DCM (6 mL). The organic layer was evaporated to dryness to give 840 mg (103%) of the methoxylated product as a yellow oil. LC-MS: t_{R} = 0.62 min, [M+1]⁺ = 290. ¹H-NMR (CDCl₃, 400MHz) δ 4.45-4.27 (m, 1 H), 3.79-3.76 (m, rotamers, 3 H), 3.59-3.38 (m, 7 H), 2.42-2.26 (m, 1 H), 2.08-2.01 (m, 1 H), 1.44 (d, *J* = 16.9 Hz), 1.48-1.40 (m, 9 H).

### Example 9: (2S,4S)-1-(tert-butoxycarbonyl)-4-hydroxy-4-(methoxymethyl)pyrrolidine-2-carboxylic acid

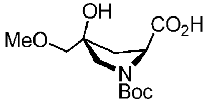

(2S,4S)-1-tert-butyl 2-methyl 4-hydroxy-4-(methoxymethyl)pyrrolidine-1,2-dicarboxylat (Example 8; 9.7 g, 33.5 mmol) was dissolved in THF (40 mL) and water (40 mL). Then lithium hydroxide monohydrate (2.8 g, 2 eq.) was added at 20-25 °C. The reaction was stirred over night at 20-25 °C. Then it was extracted with DCM (2x 50 mL). The combined organic layers were evaporated to dryness to give 3.3 g of the crude carboxylic acid as a yellow oil. The aqueous layer was acidified with aq. 1 N HCl (55 mL) and extracted with n-butanol (100 + 50 mL). The solvents were evaporated under reduced pressure at 75 °C to give 7 g of a brown foam. This second crop was a mixture of carboxylic acid and methylester. It was therefore again dissolved in THF (30 mL) and water (30 mL) and treated with lithium hydroxide (2.8 g, 2 eq.). The reaction was again stirred during 2 h at 20-25 °C. Then it was acidified with aq. 1 N HCl and extracted with n-butyronitrile (3x 35 mL). The combined organic layers were evaporated at 70 °C to dryness to give 2.6 g of a second crop of the carboxylic acid. The total yield was 5.8 g (63%). LC-MS: t_{R} = 0.58 min, [M+1]⁺ = 276. ¹H-NMR (D₆ DMSO, 400MHz) 4.15 (m, 1 H), 3.29 (m, 11 H), 2.26 (m, 1 H), 1.86 (m, 1 H), 1.37 (m, 10 H).

### Example 10: (2S,4S)-1-(tert-butoxycarbonyl)-4-hydroxy-4-(hydroxymethyl)pyrrolidine-2-carboxylic acid

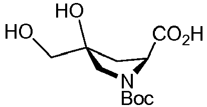

(1S,4S)-tert-butyl 1-(iodomethyl)-3-oxo-2-oxa-5-azabicyclo[2.2.1]heptane-5-carboxylate (Example 1; 6 g, 17 mmol) were dissolved in THF (18 mL) and aq. 1 M NaOH (60 mL). Then potassium acetate (3.3 g, 2 eq.) were added. The reaction was heated to 60 °C. After 1.5 h a TLC showed complete hydrolysis. After cooling down to 20-25 °C the pH was set to <1 with aq. 32% HCl (6 mL). The mixture was extracted with n-butanol (3x 80 mL). The combined organic layers were concentrated at 60 °C. After removal of the major amount of solvents coevaporation with cyclohexane (2x 120 mL) was performed until a yellow suspension was formed. This suspension was cooled to 0-5 °C. The solid was separated by filtration and was dried at 50 °C under reduced pressure to give 3.54 g of the acid as a white solid. MP =degradation above 215 °C. ¹H-NMR (D₆ DMSO, 400MHz) δ 5.77 (s, 1 H), 4.83 (s, broad, 1 H), 3.95-3.87 (m, 1 H), 3.46-3.16 (m, 4 H), 2.19-2.08 (m, 1 H), 1.50 (d, *J* = 12.8 Hz, 1 H), 1.40-1.32 (m, 9 H).

### Example 11: (5S,8S)-7-(tert-butoxycarbonyl)-2,2-dimethyl-1,3-dioxa-7-azaspiro[4.4]nonane-8-carboxylic acid

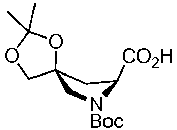

In analogy to the method described for Example 10, (1S,4S)-tert-butyl 1-(iodomethyl)-3-oxo-2-oxa-5-azabicyclo[2.2.1]heptane-5-carboxylate (Example 1; 20 g, 57 mmol) was dissolved in THF (50 mL). Then potassium acetate (11 g, 0.11 mol) was added. The resulting suspension was treated with aq. 1 N NaOH (160 mL) at 20-25 °C. After 4 h at 20-25 °C the reaction mixture was set to pH 3-4 with aq. 32% HCl (15 mL). The reaction mixture was extracted with n-butanol (3x 50 mL). The combined organic layers were washed with aq. 10% citric acid (2x 40 ml). The organic layer was evaporated at 70 °C under reduced pressure to give 19 g of a brown glass.

This crude product was dissolved in aq. 1 N NaOH (100 mL). The solution was stirred over night at 20-25 °C. The reaction was set to pH 3-4 with aq. 1 N HCl. The reaction mixture was extracted with n-butanol (3x 50 mL). The combined organic layers were evaporated under reduced pressure at 70 °C to give 14 g of a brown glass. This material was used in the next step without further purification.

(2S,4S)-1-(tert-butoxycarbonyl)-4-hydroxy-4-(hydroxymethyl)pyrrolidine-2-carboxylic acid (12.9 g, 50 mmol) was dissolved in acetone dimethylacetal (65 mL). At 20-25 °C toluolsulfonic acid (130 mg, 0.7 mmol) was added. The reaction was stirred over night at 20-25 °C. An IPC showed 94% conversion. Again toluolsulfonic acid (130 mg, 0.7 mmol) was added. After 2 h at 20-25 °C the reaction proceeded to > 99% conversion. The reaction mixture was filtered over a piece of cotton wool. The filter was washed with aceton dimethyl acetal (10 mL). Then all solvents were evaporated at ET = 60 °C to give 12.5 g of a brown sticky solid. This solid was dissolved in ethylacetate (100 mL). The solution was washed with water (100 ml). The organic layer was evaporated in vacuo at 50 °C to give 8.2 g (56%) of a beige solid. MP = 141 - 143 °C °C (degrades after melting). LC-MS: t_{R} = 0.55 min, [M-1]⁻ = 301. ¹H-NMR (D₆ DMSO, 400MHz) δ 4.19-4.14 (m, 1 H), 3.90 (s, 2 H), 3.52-3.46 (m, 1 H), 3.31 (d, *J* = 11.0 Hz, 1 H), 2.43-2.33 (m, 1 H), 2.05 (dd, *J*₁ = 5.4 Hz, *J*₂ = 13.0 Hz, 1 H), 1.44-1.42 (m, 15 H).

### Example 12: (2S,4S)-4-((benzyloxy)methyl)-1-(tert-butoxycarbonyl)-4-hydroxypyrrolidine-2-carboxylic acid

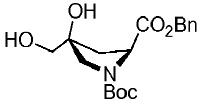

(5S,8S)-7-(tert-butoxycarbonyl)-2,2-dimethyl-1,3-dioxa-7-azaspiro[4.4]nonane-8-carboxylic acid (Example 11; 1 g, 3.3 mmol) was suspended in DCM (8.5 mL). To the suspension benzyl alcohol (435 mg, 1.2 eq.) was added at 20-25 °C. Then N,N-dicyclohexyl-carbodiimid (822 mg, 1.2 eq.) and 4-dimethylaminopyridine (20 mg, 0.05 eq.) were added. After approx. 4 h at 20-25 °C an IPC showed complete consumption of starting acid. The reaction was filtered and the filter cake was washed with DCM (3 mL). The filtrated was treated with 4 N HCl in dioxane (4 mL, 1.2 eq.) at 20-25 °C. After 2 h stirring complete cleavage of Boc and acetonide occurred. All solvents were removed in vacuo at 50 °C. The residue was take up in water and was basified with aq. 32% NaOH. Then Boc-anhydride (800 mg, 1.2 eq.) was added as a solution in THF (20 mL). After 30 min. complete consumption of free amine was detected. The mixture was extracted with TBME (2x 50 mL). The combined organic layers were evaporated and the residue was further purified by preparative HPLC to give 190 mg (16%) of the benzyl ester as a colorless oil. LC-MS: t_{R} = 0.89 min, [M+1]⁺ = 352. ¹H-NMR (D₆ DMSO, 400MHz) δ 7.39-7.24 (m, 5 H), 5.24-5.09 (m, 2 H), 4.51-4.45 (m, 1 H), 3.75 (dd, *J*₁ = 12.1 Hz, *J*₂ = 17.6 Hz, 1 H), 3.22-3.18 (m, 1 H), 2.95-2.68 (m, 3 H), 1.81-1.71 (m, 1 H), 1.41-1.24 (m, 9 H).

### Example 13: (2S,4S)-4-hydroxy-4-(methoxymethyl)-1-tosylpyrrolidine-2-carboxylic acid

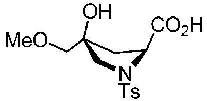

(1S,4S)-1-(iodomethyl)-5-tosyl-2-oxa-5-azabicyclo[2.2.1]heptan-3-one (Example 3; 500 mg, 1.2 mmol) were suspended in methanol (3 mL). Sodium methoxide 25% in methanol (0.6 ml, 2 eq) was added at 20-25 °C. After stirring 15 h at 20-25 °C water (1 mL) was added and the mixture was stirred during another 2 h at 20-25 °C. The reaction mixture was diluted with DCM (40 mL). The layers were separated and the pH of the aqueous layer was set to 1 with aq. 32% HCl. Then the resulting solution was extracted with DCM (2x 40 mL). The combined organic layers were evaporated to give 260 mg of a colorless oil. This oil was taken up in DMF (1 mL) and was subjected to preparative HPLC to give 120 mg (30%) of the desired acid as a white solid. MP = 132-134 °C. LC-MS: t_{R} = 0.64 min, [M+1]⁺ = 330. ¹H-NMR (D₆ DMSO, 400MHz) δ 7.72 (d, *J* = 8.2 Hz, 2 H), 7.43 (d, *J* = 8.1 Hz, 2 H), 4.20 (dd, *J*₁ = 4.5 Hz, *J*₂ = 9.0 Hz, 1 H), 3.23-3.14 (m, 2 H), 3.05 (s, 3 H), 2.96-2.87 (m, 2 H), 2.41 (s, 3 H), 2.00-1.85 (m, 2 H). ¹³C-NMR (D₆ CDCl₃, 100 MHz) δ 173 (C), 144 (C), 135 (C), 130 (CH), 128 (CH), 78 (C), 76 (CH2), 60 (CH), 59 (CH₃), 56 (CH₂), 40 (CH₂), 21 (CH₃).

### Example 14: (2S,4S)-methyl 4-hydroxy-4-(iodomethyl)-1-tosylpyrrolidine-2-carboxylate

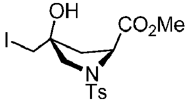

(1S,4S)-1-(iodomethyl)-5-tosyl-2-oxa-5-azabicyclo[2.2.1]heptan-3-one (Example 3; 1 g, 2.5 mmol) was dissolved in methanol (4 mL) and TBME (20 mL). Then conc. H₂SO₄ (approx. 0.1 mL) was added. The reaction was stirred during 2 days at 20-25 °C. After the first day dichloromethane (10 mL) was added to obtain a solution. After 2 days a IPC showed complete consumption of the iodo-lactone. The reaction mixture was evaporated to dryness and re-dissolved in DCM (40 mL). The solution was washed with sat. aq. NaHCO₃ soln. (40 mL) and water (40 mL). The aq. layer was extracted with DCM (40 mL). The combined organic layers were evaporated to give a beige solid which was triturated from TBME to give 0.8 g (75%) of the methyl ester as a white solid. LC-MS: t_{R} = 0.80 min, [M+1]⁺ = 440. ¹H-NMR (D₆ DMSO, 400MHz) δ 7.76 (d, *J* = 8.2 Hz, 2 H), 7.44 (d, *J* = 8.1 Hz, 2 H), 5.45 (s, 1 H), 4.56-4.51 (m, 1 H), 3.64 (s, 3 H), 3.52-3.15 (m, 4 H), 2.41 (s, 3 H), 2.11-2.06 (m, 2 H). ¹³C-NMR (D₆ DMSO, 100 MHz) δ 172 (C), 144 (C), 135 (C), 130 (CH), 128 (CH), 77 (C), 61 (CH₂), 58 (CH₂), 53 (CH₃), 42 (CH₂), 22 (CH₃), 16 (CH₂).

### Example 15: (2S,4S)-benzyl 4-hydroxy-2-(hydroxymethyl)-4-methylpyrrolidine-1-carboxylate

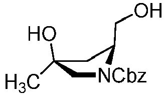

(1S,4S)-benzyl 1-(iodomethyl)-3-oxo-2-oxa-5-azabicyclo[2.2.1]heptane-5-carboxylate (Example 4; 150 mg, 0.57 mmol) was dissolved in DMSO (1.5 mL). The resulting solution was treated with sodium borohydride (32 mg, 1.5 eq.) at 20-25 °C. The mixture was heated up to 100 °C. After 4 h stirring at 100 °C the starting material was completely consumed. After cooling down to 20-25 °C the reaction was diluted with iPrOAc (50 mL). The mixture was washed with aq. 4% NaHSO₄ soln. (2x 50 mL) and water (2x 50 mL). All solvents were removed under reduced pressure at 50 °C to give 90 mg of crude product. This was further purified by preparative HPLC. The fractions containing the product were pooled and evaporated by lyophilisation to give 40 mg of the alcohol as a colourless oil. LC-MS: t_{R} = 0.65 min, [M+1]⁺ = 266. ¹H-NMR (D₆ DMSO, 400MHz) δ 7.39-7.30 (m, 5 H), 5.19-4.99 (m, 4 H), 3.85-3.81 (m, 1 H), 3.72-3.65 (m, 1 H), 3.56-3.50 (m, 1 H), 3.36-3.23 (m, 4 H), 1.98-1.92 (m, 2 H), 1.23 (s, 3 H).

### Example 16: (2S,4R)-1-tert-butyl 2-methyl 4-((1,3-dioxoisoindolin-2-yl)methyl)-4-hydroxypyrrolidine-1,2-dicarboxylate

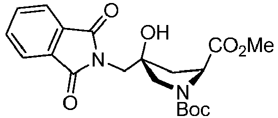

(2S,4S)-1-tert-butyl 2-methyl 4-hydroxy-4-(iodomethyl)pyrrolidine-1,2-dicarboxylate (Example 7; 300 mg, 0.8 mmol) was dissolved in DMF (1.5 mL). The solution was treated with phtalimide potassium salt (159 mg, 1.1 eq) at 20-25 °C. After 4 days of stirring another amount of phtalimide potassium salt (144 mg, 1.0 eq) was added. After 6 days at 20-25 °C the reaction was diluted with iPrOAc (50 mL). The mixture was washed with water (3x 40 mL). The organic layer was evaporated to dryness to give 270 mg of crude product which was purified by preparative HPLC to give 128 mg (41%) of pure phtalimide as a white solid. MP = 128-129 °C. LC-MS: t_{R} = 0.79 min, [M+1]⁺ = 405. ¹H-NMR (D₆ DMSO, 400MHz) δ 7.9-7.85 (m, 4 H), 5.19 (s, broad, 1 H), 3.72-3.58 (m, 4 H), 3.50-3.26 (m, 4 H), 2.38-2.34 (m, 1 H), 1.95-1.87 (m, 1 H), 1.46-1.24 (m, 9 H).

### Example 17: (2S,4R)-methyl 4-((1,3-dioxoisoindolin-2-yl)methyl)-4-hydroxy-1-tosylpyrrolidine-2-carboxylate

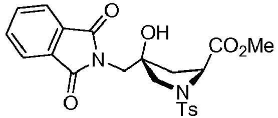

(2S,4S)-methyl 4-hydroxy-4-(iodomethyl)-1-tosylpyrrolidine-2-carboxylate (Example 14; 250 mg, 0.6 mmol) were dissolved in DMF (1 mL). The solution was treated with phtalimide potassium salt (220 mg, 2.1 eq). An IPC (LC-MS) after 24 h at 20-25 °C showed >90% conversion. The reaction was diluted with iPrOAc (50 mL). The mixture was washed with water (3x 40 mL). The organic layer was evaporated to dryness. The crude product was purified by preparative HPLC to give 200 mg (77%) of the phtalimide as a white foam. LC-MS: t_{R} = 0.81 min, [M+1]⁺ = 459. ¹H-NMR (D₆ DMSO, 400MHz) δ 7.91-7.87 (m, 4 H), 7.65 (d, *J* = 8.2 Hz, 2 H), 7.16 (d, *J* = 8.1 Hz, 2 H), 5.37 (s, 1 H), 4.55 (d, *J* = 8.8 Hz, 1 H), 3.65-3.62 (m, 3 H), 3.22-3.22 (m, 1 H), 3.10 (d, *J* = 10.8 Hz, 1 H), 2.15 (s, 3 H), 2.01 (d, *J* = 13.1 Hz, 1 H), 1.59 (dd, *J*₁ = 9.5 Hz, *J* ₂ = 13.1 Hz, 1 H). ¹³C-NMR (D₆ DMSO, 100 MHz) δ 172 (C), 169 (C), 144 (C), 135 (C); 135 (CH), 132 (CH), 130 (CH), 128 (C), 124 (CH), 79 (C), 61 (CH), 58 (CH₂), 52 (CH₃), 43 (CH₂), 40 (CH₂), 21 (CH₃).

### Example 18: (2S,4R)-4-((benzylamino)methyl)-1-(tert-butoxycarbonyl)-4-hydroxypyrrolidine-2-carboxylic acid

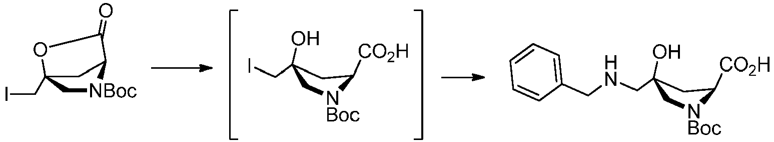

(1S,4S)-tert-butyl 1-(iodomethyl)-3-oxo-2-oxa-5-azabicyclo[2.2.1]heptane-5-carboxylate (Example 1; 0.5 g, 1.4 mmol) was dissolved in THF (3 mL). The solution was treated with aq. 1 N NaOH (6 mL). After 20 min at 20-25 °C the resulting emulsion was transformed into a solution. Then benzylamine (0.15 g, 1.4 mmol) was added at 20-25 °C. The mixture was stirred during 2 h at 20-25 °C. Then an IPC revealed complete conversion to product. The reaction was washed with heptanes (1x 10 mL). Then the pH was set to 3 with sat. aq. 32% HCl. The reaction was extracted with DCM (1x 10 mL). The organic layer was evaporated in vacuo at 50 °C. The residue was taken up in DCM (10 mL). The resulting suspension was filtered through cellite. The filtrate was evaporated to give 320 mg (65%) of the product as a yellow glass. LC-MS: t_{R} = 0.72 min, [M]⁺ = 349. ¹H-NMR (D₃COD, 400MHz) δ 7.54-7.46 (m,. 5 H), 4.75 (s, 2 H), 3.66-3.61 (m, 1 H), 3.40-3.33 (m, 4 H), 3.14-3.1 (m, 2 H), 2.30-2.21 (m, 1 H), 2.05 -2.01 (m, 1H), 1.49-1.42(m, 9H).

### Example 19: (2S,4R)-4-(aminomethyl)-4-hydroxy-1-tosylpyrrolidine-2-carboxylic acid

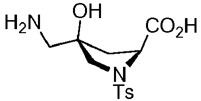

(1S,4S)-1-(iodomethyl)-5-tosyl-2-oxa-5-azabicyclo[2.2.1]heptan-3-one (Example 3; 500 mg, 1.2 mmol) was dissolved in THF (0.5 mL). Then aq. 25% NH₄OH solution was added at 20-25 °C. After stirring 40 min at 20-25 °C a brown solution was obtained and an IPC showed complete conversion to the amine. The reaction was diluted with water (25 mL) and was extracted with DCM (2x 20 mL). The aq. layer was concentrated by lyophilisation to dryness to give 460 mg (119 %) of a beige foam. LC-MS: t_{R} = 0.65 min, [M+ACN]⁺ = 355. ¹H-NMR (D₆ DMSO, 400MHz) δ 7.75 (d, *J* = 7.9 Hz, 2 H), 7.54-7.45 (m, 2 H), 4.08 (dd, *J*₁ = 1.9 Hz, *J*₂ = 9.3 Hz, 1 H), 3.37 (d, *J* = 9.9 Hz, 1 H), 3.09 (d, *J* = 9.9 Hz, 1 H), 2.67 (s, 2 H), 2.43 (s, 3 H), 1.98-1.84 (m, 2 H). ¹³C-NMR (D₆ DMSO, 100 MHz) δ 175 (C), 144 (C), 134 (C), 130 (CH), 128 (CH), 77 (C), 61 (CH), 59 (CH₂), 45 (CH₂), 40 (CH₂), 22 (CH₃).

### Example 20: (2S,4S)-tert-butyl 4-hydroxy-2-(hydroxymethyl)-4-(iodomethyl)pyrrolidine-1-carboxylate

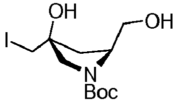

(1S,4S)-tert-butyl 1-(iodomethyl)-3-oxo-2-oxa-5-azabicyclo[2.2.1]heptane-5-carboxylate (Example 1, 19.9 g, 56 mmol) was dissolved THF (120 ml). The solution was cooled to 0-5 °C. Then LiBH₄ (28 ml, 2 M in THF, 56 mmol) was added at 0-15 °C. After 40 min stirring at 0-5 °C an IPC showed complete consumption of starting material. The reaction was carefully quenched sequentially with acetone (11 ml, 153 mmol) and aq. 10% citric acid (100 ml). The mixture was extracted with EtOAc (100 ml). The aq. layer was extracted with ethylacetate. The organic layers were washed with sat. aq. NaHCO₃ solution (100 ml) and water (100 ml). Then all solvents were removed on a rotavapor under vacuum and 50 °C to give 19.5 g (97%) of the alcohol as a yellow oil.

¹H-NMR (D₆DMSO, 400MHz) δ 5.4 (s, 1 H), 5.05-5.0 (m, 1 H), 3.80-3.70 (m, 1 H), 3.60-3.10 (m, 6 H), 2.10-1.90 (m, 2 H), 1.41 (s, 9 H).

## Claims

1. A process for the synthesis of 1-(halo-methyl)-3-oxo-2-oxa-5-azabicyclo[2.2.1]heptane derivatives of the formula (II) 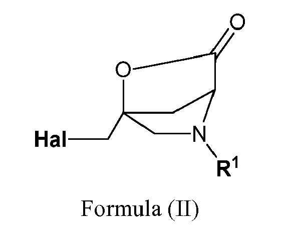 said process comprising the halo-lactonization of a compound of formula (I) 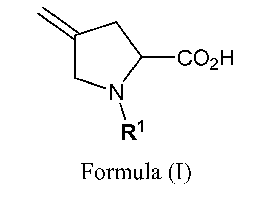 wherein
**Hal** represents iodine or bromine; and
**R¹** represents a nitrogen protecting group.

2. A process according to claim 1, wherein said process is performed starting from the enantiomerically enriched L- or D- proline derivative of formula (Ia), respectively formula (Ib)

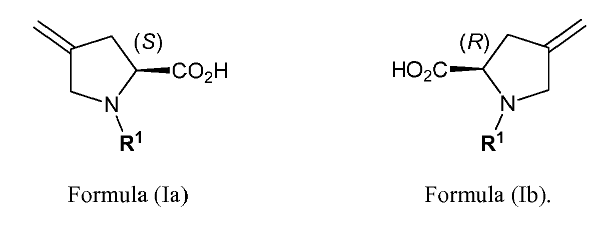

3. A process according to claims 1 or 2, wherein said halo-lactonization is performed in presence of a solvent selected from DMF, water, or a mixture thereof.

4. A process according to claims 1 to 3, wherein said halo-lactonization is performed in presence of a base.

5. A process according to claim 4, wherein said base is a alkali metal carbonate solution, or an alkali metal hydrogen carbonate solution.

6. A process according to any one of claims 1 to 5, wherein the compound of the formula (II) is further transformed to a compound of the formula (III): 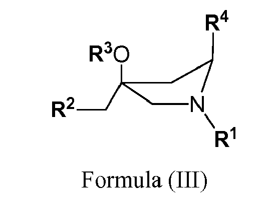 wherein
**R¹** represents a nitrogen protecting group;
**R²** represents hydrogen, iodine, bromine, hydroxy, alkoxy, or an optionally substituted amino group;
**R³** represents hydrogen;
**R⁴** represents -CH₂-OH; or COO-**R⁴¹**, wherein **R⁴¹** represents hydrogen, C₁-₈-alkyl, C₁-₃-fluroroalkyl, or optionally substituted aryl-C₁-₆-alkyl.

7. A process according to claim 6, wherein said transformation of the compound of formula (II) to the compound of formula (III) comprises a ring opening reaction of the lactone ring of the compounds of formula (II); and wherein said ring opening of the lactone ring of the compounds of formula (II) is achieved under acid catalysis; or said ring opening of the lactone ring of the compounds of formula (II) is achieved by nucleophilic attack under neutral or basic conditions.

8. A process according to claim 7, wherein said process is performed in presence of a solvent selected from an alcohol, an aromatic solvent; an ether solvent; a chlorinated organic solvent, an aprotic polar solvent, water; or a mixture thereof.

9. A compound of the formula (II): 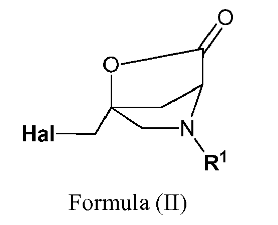 wherein
**R¹** represents a nitrogen protecting group; and
**Hal** represents bromine, or especially iodine.

10. A compound according to claim 9, wherein said compound is:
(1S,4S)-tert-butyl-1-(iodomethyl)-3-oxo-2-oxa-5-azabicyclo[2.2.1]heptane-5 carboxylate;
(1S,4S)-tert-butyl-1-(bromomethyl)-3-oxo-2-oxa-5-azabicyclo[2.2.1]heptane-5-carboxylate;
(1S,4S)-1-(iodomethyl)-5-tosyl-2-oxa-5-azabicyclo[2.2.1]heptan-3-one;
(1S,4S)-benzyl-1-(iodomethyl)-3-oxo-2-oxa-5-azabicyclo[2.2.1]heptane-5-carboxylate;
(1S,4S)-5-benzoyl-1-(iodomethyl)-2-oxa-5-azabicyclo[2.2.1]heptan-3-one; or
(1S,4S)-1-(iodomethyl)-5-(2-nitrophenyl)-2-oxa-5-azabicyclo[2.2.1]heptan-3-one.

11. A compound of formula (III) as defined for the process of claim 6, wherein said compound is:
(2S,4S)-1-(tert-butoxycarbonyl)-4-hydroxy-4-(methoxymethyl)-pyrrolidine-2-carboxylic acid;
(2S,4S)-1-(tert-butoxycarbonyl)-4-hydroxy-4-(hydroxymethyl)-pyrrolidine-2-carboxylic acid;
(2S,4S)-4-hydroxy-4-(methoxymethyl)-1-tosylpyrrolidine-2-carboxylic acid;
(2S,4R)-4-((benzylamino)methyl)-1-(tert-butoxycarbonyl)-4-hydroxypyrrolidine-2-carboxylic acid;
(2S,4R)-4-(aminomethyl)-4-hydroxy-1-tosylpyrrolidine-2-carboxylic acid;
(2S,4S)-methyl 4-hydroxy-4-(iodomethyl)-1-tosyl-pyrrolidine-2-carboxylate;
(2S,4R)-1-tert-butyl-2-methyl-4-((1,3-dioxoisoindolin-2-yl)methyl)-4-hydroxypyrrolidine-1,2-dicarboxylate;
(2S,4R)-methyl-4-((1,3-dioxoisoindolin-2-yl)methyl)-4-hydroxy-1-tosylpyrrolidine-2-carboxylate;
(2S,4S)-benzyl-4-hydroxy-2-(hydroxymethyl)-4-methyl-pyrrolidine-1-carboxylate; or
(2S,4S)-tert-butyl-4-hydroxy-2-(hydroxymethyl)-4-(iodomethyl)-pyrrolidine-1-carboxylate.

12. The compound (5S,8S)-7-(tert-butoxycarbonyl)-2,2-dimethyl-1,3-dioxa-7-azaspiro[4.4]nonane-8-carboxylic acid.
